# EUROPEAN PATENT APPLICATION

(11) **EP 2 535 076 A1**
(43) Date of publication of application: **19.12.2012**
(21) Application number: 11425158.0
(22) Date of filing: 14.06.2011
(51) Int. Cl.: A61M 16/04, A61B 1/267

(54) **Device supporting a tracheal tube**

(71) Applicant: De Domenico, Andrea, 00176 Roma (IT)
(72) Inventor: De Domenico, Andrea, 00176 Roma (IT)
(74) Representative: Zizzari, Massimo

(57) **Abstract**

Device supporting a tracheal tube, preferably applied to a blade (104) of a laryngoscope, or an *Airtraq® videolaryngoscope* (100), or a *LMA® videolaryngoscope*, or other similar instruments, particularly suitable as a support to procedure of patient's tracheal intubation, and wherein it comprises:
- one or more planar elements (151), (152), each of them having a profile that is perfectly adapted to the curvature of said blade (104) in which it is placed, and from which it can protrude out, on command, through a longitudinal groove, in order to achieve one or more fixed transversal walls, that are continuous or in patches; a tracheal tube (108) that goes with its far end (110) across said one or more transversal walls, and that can be curved and addressed towards trachea of a patient;
- a set of mechanical gears that: according to a back position of a sliding button (153), keep said one or more planar elements (151), (152), placed inside the above blade (104); and that according to a front position of the same sliding button (153), push outside and distally the above said one or more planar elements (151), (152), in order to achieve said one or more fixed transversal walls.

## Description

The present invention concerns a medical device, preferably applied to a blade of a laryngoscope, or an *Airtraq® videolaryngoscope,* or a *LMA*® *videolaryngoscope,* or other similar instruments, particularly suitable as a support to procedure of patient's tracheal intubation. In particular, the device includes one or more planar elements, each having a profile that is perfectly adapted to the curvature of said blade in which it is placed, and from which it can protrude out, on command, through a groove, in order to achieve one or more respective fixed transversal walls.

A tracheal tube that goes with its far end across said one or more transversal walls, can be curved and addressed towards a trachea of a patient.

The direct laryngoscopy is a technique that permits to visualize the laryngeal structures in a clear and straight way, and it is achieved by an instrument called laryngoscope. This technique made it possible, to address to anesthetists operating around the world, to define a standard procedure of tracheal intubation in order to place a tube in trachea, by which a patient can receive oxygen and at the same time can have protected air ways during the anesthesia.

Despite the improvement of such technique, obtained at a global level by years of clinical practice, and despite the different shapes of laryngoscopic blades that have been designed and are available on the market, in same cases the direct laryngoscopy is not possible, because of particular anatomical parts in some persons, or because of particular situations that can happen in emergency.

In example, it is not possible when the alignment of the three patient's axis (oral axis, pharyngeal axis and laryngeal axis) cannot be achieved by the standard actions that a physician knows and can do. Therefore, since a long time there was a widely recognized need to find some alternative instruments and techniques, so that an optimal visualization of glottis can be achieved, even in case that direct laryngoscopy results not effective or either impossible.

When new technologies become available, with new advanced materials, the laryngeal structures had more chances to be properly visualized, even in a not direct way, by the so called indirect laryngoscopy.

Therefore, the techniques of indirect laryngoscopy made it possible to visualize the laryngeal structures clearly, using a camera, or an optical fiber, or further a system of prisms with the distal elements that can be placed close to the glottis.

Nowadays, a large set of instruments are available, permitting to handle even hard situations, and many of these instruments embed indirect laryngoscopy based systems.

With reference to devices that, at the present time, permit to achieve the indirect laryngoscopy technique, except the flexible and stiff fiberscope, or *Bonfils* fiberscope, they belong to two possible classes:
- the videolaryngoscopes (Glidescope, McGrath, and C-MAC);
- the tunneled systems (Upscher Scope, *Bullard laryngoscope,* Airtraq, Pentax AWS, and A.P. Advance LMA).

Besides the different visual, optical or video system, each device belongs to the first or the second class, according to the procedure of use of the same device, when the operator can see the laryngeal structures and can insert the endotracheal tube up to its proper position in trachea.

Devices in the first class, *Glidescope, McGrath,* and *C-MAC,* once the indirect laryngoscopy is possible, because of the blade's shape, usually require that the operator, before to insert the endotracheal tube, has previously and properly shaped it. Therefore, the tube's profile is adapted, according to a specific angle, by inserting a stiff stylet inside, so that the final shape allows to route the tube beyond the tongue, towards the patient's trachea.

Instead, devices in the second class, like i.e. *Airtraq, Pentax AWS,* and *A.P. Advance LMA,* contain the endotracheal tube in a tunnel that is integrated into the blade, so that a guidance can be achieved all along the path, until the tube exits with the far end directly on the proper position in trachea. Therefore, the procedure does not require the operator to use a stylet, and to maneuver the endotracheal tube either, it just requires the tube to be pushed inside a stiff routing channel.

Although this solution prevents possible risks arising from use of stylets, and therefore represents a more comfortable procedure of orotracheal intubation, according to an indirect laryngoscopy technique, however it is characterized by significant drawbacks. First of all, the operator is forced to express strong actions of pulling and rotating the device, just as an attempt, sometimes not effective, to align properly the far end of the instrument to the tracheal axis.

Furthermore, the requirement to insert the endotracheal tube in an operative channel, in order to follow a proper routing path, leads as a consequence to the definition of an increased instrument's thickness, and to an increased minimum inter-foreteeth distance, so that the same instrument can enter into the patient's mouth, and/or leads to the use of different instruments of different size, according to the diameter of the same tube to be inserted.

Therefore, the subject of the present invention consists of a device overcoming all the previous drawbacks, and achieving a great versatility of use, because it can be embedded into large part of the available systems supporting the orotracheal intubation, actually on the market, like i.e.: laryngoscopes, videolaryngoscopes, tunneled videolaryngoscopes, etc.

In particular, the device of this invention achieves a guiding and routing system for an endotracheal tube by use of one or more planar elements, each having a profile that is perfectly adapted to the curvature of said blade in which it is placed, and from which it can protrude out, on command, through a groove, in order to achieve one or more respective fixed transversal wall.

Another significant feature of the present invention is the possibility of integration in a guiding system by magnetic means, according to concepts and disclosure from the European patent application EP 11425045 (De Domenico), so that the intubation procedure can be adapted to different operative circumstances, especially in case where a direct or indirect visual perception of the situation is possible. More specifically, the above integration would achieve a synergy of the two systems, the first based on planar elements and the second based on magnetic elements, so that a smaller size of both devices is possible, according to smaller planar elements and smaller magnetic elements either, without affecting the effectiveness of instruments, and improving instead the general efficiency due to the decreased length of inter-foreteeth distance.

The disclosed device permits therefore to achieve a significant decrease of thickness, a variable routing direction at each step of the procedure, under control of a guiding mechanism, a more comfortable intubation that does not require any use of a stylet to define shape and profile.

Therefore, it is specific subject of the present invention a device supporting a tracheal tube, preferably applied to a blade of a laryngoscope, or an *Airtraq*® *videolaryngoscope,* or a *LMA*® *videolaryngoscope,* or other similar instruments, particularly suitable as a support to procedure of patient's tracheal intubation, and characterized in that comprising:
- one or more planar elements, each of them having a profile that is perfectly adapted to the curvature of said blade in which it is placed, and from which it can protrude out, on command, through a longitudinal groove, in order to achieve one or more fixed transversal walls, that are continuous or in patches; a tracheal tube that goes with its far end across said one or more transversal walls, and that can be curved and addressed towards trachea of a patient;
- a set of mechanical gears that: according to a back position of a sliding button, keep said one or more planar elements placed inside the above blade; and that according to a front position of the same sliding button, push outside and distally the above said one or more planar elements, in order to achieve said one or more fixed transversal walls.

The present invention will now be described for illustrative but not limitative purposes, according to its preferred embodiments, with particular reference to figures of the enclosed drawings, wherein:
figure 1 is a front perspective view of a device supporting a tracheal tube, according to the present invention, preferably embedded in a *Airtraq*® *videolaryngoscope;*
figure 2 is a lateral view of a device supporting a tracheal tube, embedded in a *Airtraq*® *videolaryngoscope,* like that of figure 1, inserted in the upper part of a patient, related to mouth and first tract of air ways, the same patient being represented according to a lateral sectioned view;
figures 3, 4 and 5 are a sequence of front perspective views of a *Airtraq*® *videolaryngoscope,* similar to that of figure 1, further including a set of magnetic elements, and of an endotracheal tube that is moved in contact with the same magnetic elements, that is represented at three different positions;
figures 6, 8 and 10 are a sequence of front perspective views, in transparency, of a sectioned part of the blade of a *Airtraq*® *videolaryngoscope* like that of figure 1, where the inner components are protruded outside, and where they are represented at three different positions;
figures 7, 9 and 11 are a sequence of front perspective views, in solid, of a sectioned part of the same blade of a *Airtraq*® *videolaryngoscope* like that of figure 1, where the inner components are protruded outside, and distally, and where they are represented at three different positions;
figure 12 is a front perspective view, in transparency, of an alternative embodiment of the present invention, again applied to a sectioned part of the same blade of a *Airtraq*® *videolaryngoscope* like that of figure 1, where components have been moved outside, and their run has been stopped by a set of inner small cylindrical and transversal elements;
figure 13 is a front perspective view, in solid, of the same object represented in figure 12;
figure 14 is a front perspective view, in transparency, of an alternative embodiment of the present invention, again applied to a sectioned part of the same blade of a *Airtraq*® *videolaryngoscope* like that of figure 1, where components have been moved outside, and they are represented by a unique spoon-shaped convex item, and their run has been stopped by a set of inner small cylindrical and transversal elements;
figure 15 is a front perspective view, in solid, of the same object represented in figure 14;
figure 16 is a front perspective view, in solid, of the same object represented in figure 14, and of a tracheal tube that goes with its far end across said spoon-shaped item, so that said tube can be curved and addressed towards trachea of a patient;
figure 17 is a front perspective view of a device supporting a tracheal tube, according to the present invention, preferably embedded in a *LMA*® *videolaryngoscope* with magnetic elements;
figure 18 is a rear perspective view of a device supporting a tracheal tube, according to the present invention, preferably embedded in the same *LMA*® *videolaryngoscope* with magnetic elements;
figure 19 is a lateral view of a device supporting a tracheal tube, applied to the same *LMA*® *videolaryngoscope* with magnetic elements, like that of figures 17 and 18, inserted in the upper part of a patient, related to mouth and first tract of air ways, the same patient being represented according to a lateral sectioned view;
figure 20 is a front perspective view of a device supporting a tracheal tube, according to an alternative embodiment, comprising two respective service channels: one on the right and one on the left;
figure 21 is a front view of a device supporting a tracheal tube, like that of figure 20, comprising two respective service channels: one on the right and one on the left.

It is here underlined that only few of the many conceivable embodiments of the present invention are described, which are just some specific non-limiting examples, having the possibility to describe many other embodiments based on the disclosed technical solutions of the present invention.

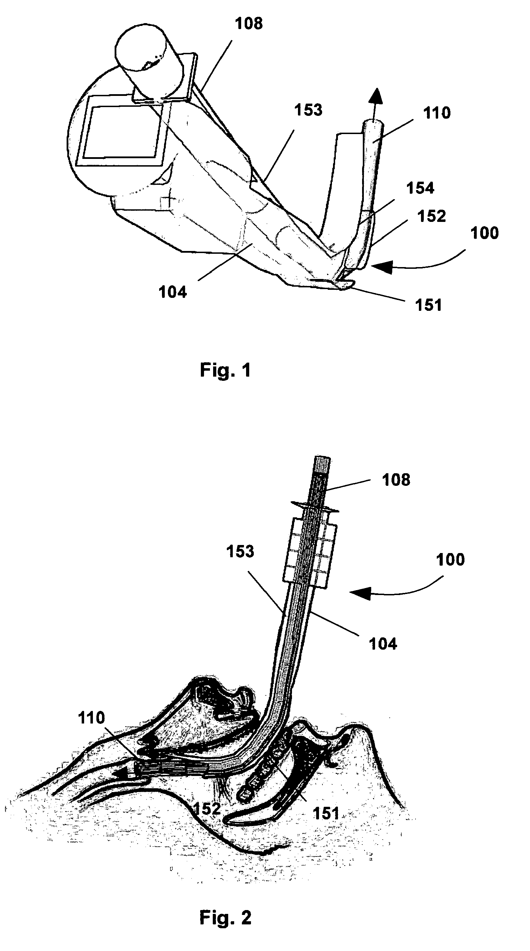

In figure 1 it is illustrated a *Airtraq*® *videolaryngoscope* 100, particularly suitable as a support to the procedure of tracheal intubation of a patient. The blade 104, of the same *videolaryngoscope* 100, in the prior art has a longitudinal profile with some fixed protruding walls 151, 152, 154, that achieve a guiding and routing *tunnel,* along which an endotracheal tube 108 is inserted up to its final position, with the distal end in trachea, as illustrated in figure 2.

Instead, device 100 of the present invention is characterized by having the protruding walls partially or completely removed. More exactly, the upper wall 154 has been partially removed, so that it can still push the upper tissues and tongue of patient, instead the lower wall has been completely removed. As a consequence of having this new geometrical shape, the device 100 results to be smaller, and its insertion in the first tract of patient's air ways results to be more comfortable to medical doctors, and at the same time it is decreased the risk of wrong and/or traumatic insertions.

Indeed, when device 100 is placed at its final position, the function guiding a tracheal tube 108 is achieved again, by a mobile protruding wall 151, 152, that is pushed outside and forward, through a longitudinal groove of blade 104. The wall is composed by one or more planar elements 151, 152, each of them having a profile that is perfectly adapted to the curvature of said blade in which it is placed. For this reason, elements 151, 152, are preferably made using a material, or a metallic alloy, that is highly elastic and strong, like in example a flexible steel having a not traumatic edge. The mechanism providing a push outside, on command, of elements 151, 152, through a longitudinal groove of blade 104, includes a sliding button 153, preferably placed in comfortable position to doctors, that is in the upper part of device 100. In such a way, once the procedure of insertion of tracheal tube 108 is started, following a lateral sliding path along the device 100, it is possible to activate said mechanism that pushes outside, simultaneously or in sequence, and forward, one or more elements 151, 152, in such a way to enable to "play off the cushion", because the far end 110 can be curved upwards and can be addressed to trachea of a patient. In particular, the mechanism of pushing outside and forward, on command, includes a set of mechanical gears that: according to a back position of a sliding button 153, keep said one or more planar elements 151, 152, placed inside the above blade 104; and that according to a front position of the same sliding button 153, push outside and forward the above said one or more planar elements 151, 152, outside and distally, in order to achieve the fixed transversal wall.

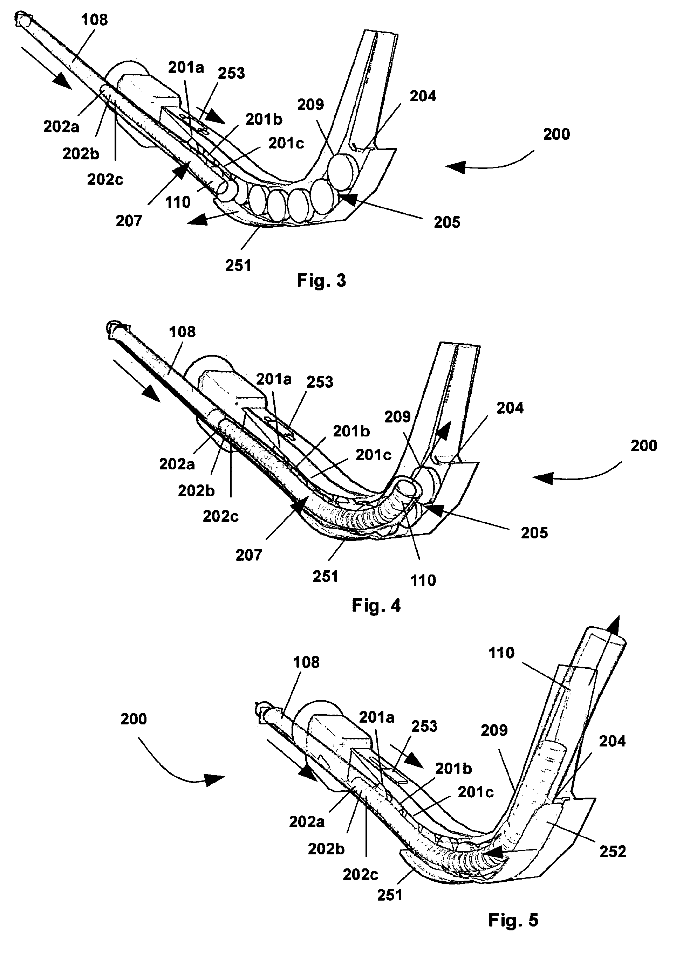

Figures 3, 4 and 5 are a sequence of front perspective views of a *Airtraq*® *videolaryngoscope* 200, similar to that of figure 1, further including a set of magnetic elements, according to concepts and disclosures from the European patent application EP 11425045 (De Domenico), filed the 23 February 2011.

In device 200 the guiding function is achieved through a *magnetic platform* 205, that is placed into the blade, and that interacts with a *magnetic train* 207, that has been previously and steadily inserted within an endotracheal tube 108.

The *magnetic platform* 205 comprises a first set of magnetic elements 201 a, 201 b, ..., etc., that are all connected each other, by a first flexible supporting structure; the *magnetic train* 207 comprises instead a second set of magnetic elements 202a, 202b, ..., etc., that are all connected each other, by a second flexible supporting structure. The elements 202a, 202b, ..., etc., of the *magnetic train* 207 have an opposite polarity in respect of elements 201 a, 201 b, ... , etc., of said *magnetic platform* 205, so that a magnetic attractive force is generated by interaction all the time, keeping said endotracheal tube 108 strictly in contact with said *magnetic platform* 205.

Therefore, figures 3, 4 and 5 show a sequence where an endotracheal tube 108 is moved in contact with the *magnetic platform* 205, and it is represented at three different positions.

An operator can move the endotracheal tube 108, forward or backward, along the longitudinal profile of said operative channel 204, in order to achieve a guiding and routing system of the same endotracheal tube 108, because the tube follows a respective curved trajectory and it extends itself with its far end 110 aligned to a direction that follows the tangent to said operative channel's 204 profile, at the last point of magnetic contact 209.

Therefore, the guiding system of the present invention is integrated in said magnetic guiding system, already known from the prior art, and it includes a mobile protruding wall 251, 252, that is pushed outside, through a longitudinal groove, from the blade of the same device 200.

In figure 3 it is shown that a forward motion of sliding button 253 activates a mechanism, which is smooth or by click, pushing outside first the protruding element 251, like in figure 4, and then the protruding element 252, like in figure 5. In such a way, elements 251, 252, enable the tracheal tube 108 to "play off the cushion", because the far end 110 can be curved upwards and can be addressed to trachea of a patient.

Said mechanism pushing outside each protruding element is explained now more in detail, with reference to figures 3, 4 and 5.

The object shown in the same figures 3, 4 and 5 is a sectioned part of the blade 606 of a *Airtraq*® *videolaryngoscope,* or other similar instruments, having a squared cross-section, with a hollow inside, where a triangle-based support 605 is housed. This support 605 can move laterally outside of blade 606, following a motion transmitted by a rod 601 connected to it. In the upper part, the same rod 601 is connected to a sliding button (not illustrated).

A motion forward of said button causes the motion forward of rod 601, following the direction of arrow and, through an articulated tract 603 and pivots 602 and 604, the same motion is transmitted to support 605, sliding outside of blade 606, along a lateral oblique wall, in touch to another triangle-based and fixed element 610. A protruding element 607 is connected on the top of said support 605, and represents a wall that, touched by the far end of a tracheal tube, enables it to "play off the cushion", changing and curving the insertion trajectory of the same tracheal tube.

The protruding element 607 has a transversal edge 609 on the inner side, that is intercepted by a respective edge 608 on the profile of blade 606, so that an end-run function is embedded in motion of said element 607.

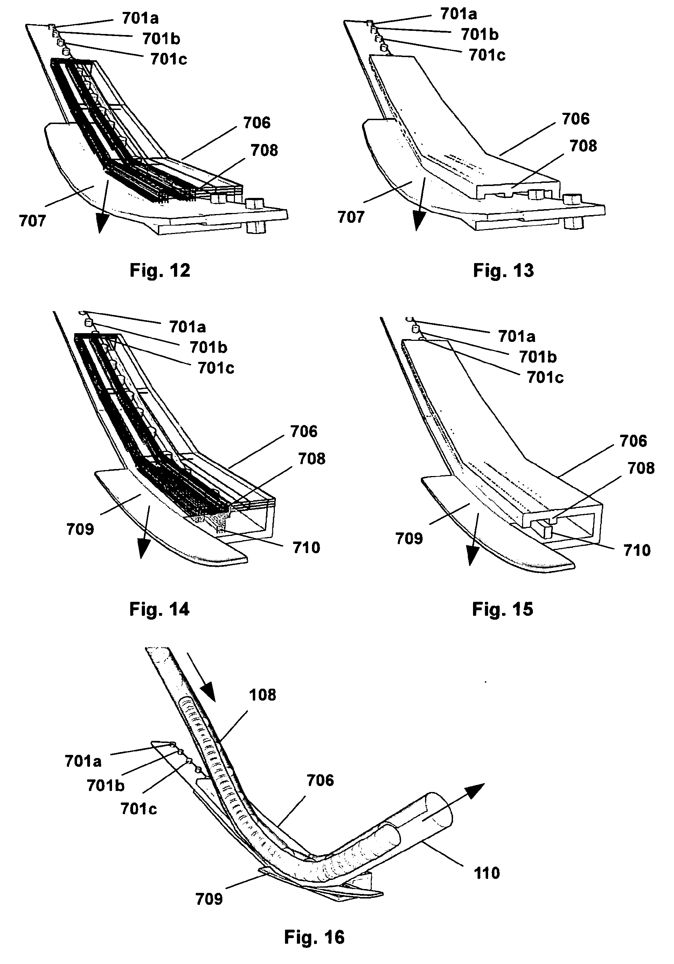

Figure 16 shows more in detail how a far end 110 of a tracheal tube 108, that touches element 709, causes its trajectory to be curved upwards.

The above described mechanism allows, therefore, a more comfortable and effective maneuvering to doctors, in order to achieve a proper tracheal intubation of patients.

The mechanism pushing outside the protruding element 607 is perfectly reversible, and that means that a motion backward of said sliding button causes a motion backward of said rod 601 following a direction opposite to arrow and, through the same articulated tract 603 and pivots 602 and 604, the motion is transmitted to support 605, sliding inside of blade 606, along a lateral oblique wall, in touch to the triangle-based and fixed element 610.

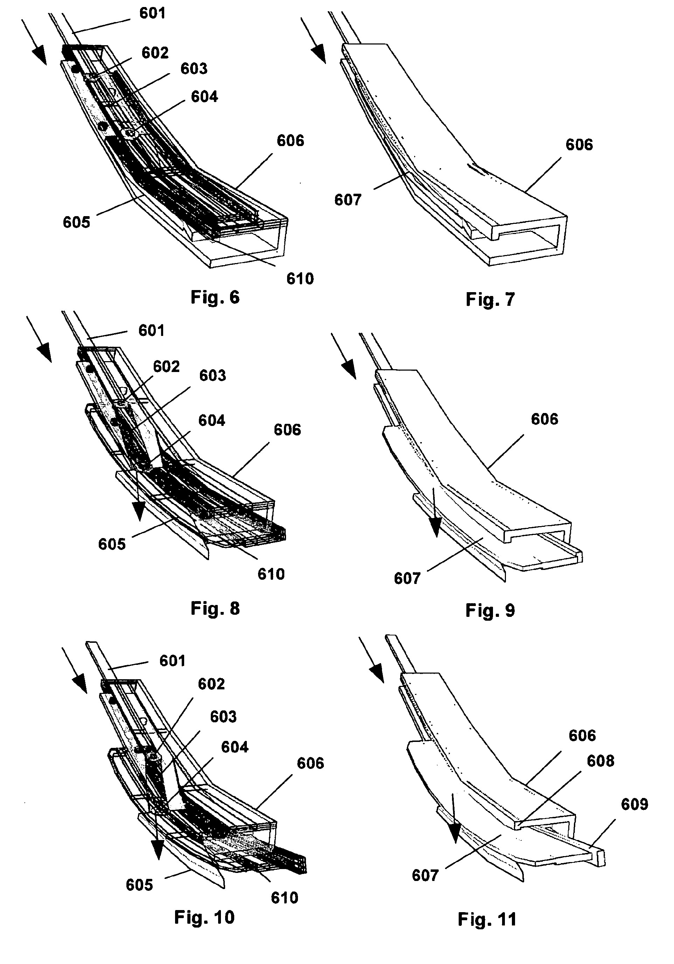

Figures 7, 9 and 11 show the same elements and mechanisms, in solid, so that a condition of real use of device is represented.

The described mechanism can include, without loss of generality, more protruding elements, in example two or three, that are pushed outside, on command, from the blade of device, sequentially or simultaneously, and that are installed at different distances, along the longitudinal axis of the blade. Furthermore, said protruding elements can lie on different levels, one in respect to the others, and can possibly being placed with a certain degree of overlap. Again, the same protruding elements can be installed on different supporting structures, in example: one set on the right side and another set on the left side in respect to a channel where a tracheal tube is inserted.

Figure 12 shows, in transparency, an alternative embodiment of the present invention, applied to a blade 706, where the protruding element 707 is composed, indeed, by a unique flexible plate, having a set of small cylinders 701 a, 701 b, 701 c, etc., installed on it following a longitudinal line, so that when they intercept the edge 708 embed the same function of end-run, as previously described.

Figure 14 shows, in transparency, another alternative embodiment of the present invention, similar to the previous one, where components 709 are represented by a unique spoon-shaped convex item. Again, a set of small cylinders 701 a, 701 b, 701c, etc., are installed on it following a longitudinal line, and when they intercept the edges 708 and 710 embed the same function of end-run, as previously described.

Figures 13 and 15 show the same elements and mechanisms, in solid, so that a condition of real use of device is represented.

The mechanism controlling the above action of pushing outside the protruding elements, or pulling them inside the blade, and/or their simultaneous motion forward or backward along the longitudinal axis of the same blade, can further include additional gears, defining and enabling a "click" mechanism. These additional gears can include springs that are charged, with stopping clips that, when removed by a command button, cause the protruding wall to be immediately "clicked" outside, or "clicked" inside.

Further embodiments of the invention can include more mechanisms, one for each protruding element, that can be activated simultaneously, or sequentially, and the related technical changes are immediate to those skilled in the art, in order to obtain medical devices that are comfortable and easy to be used.

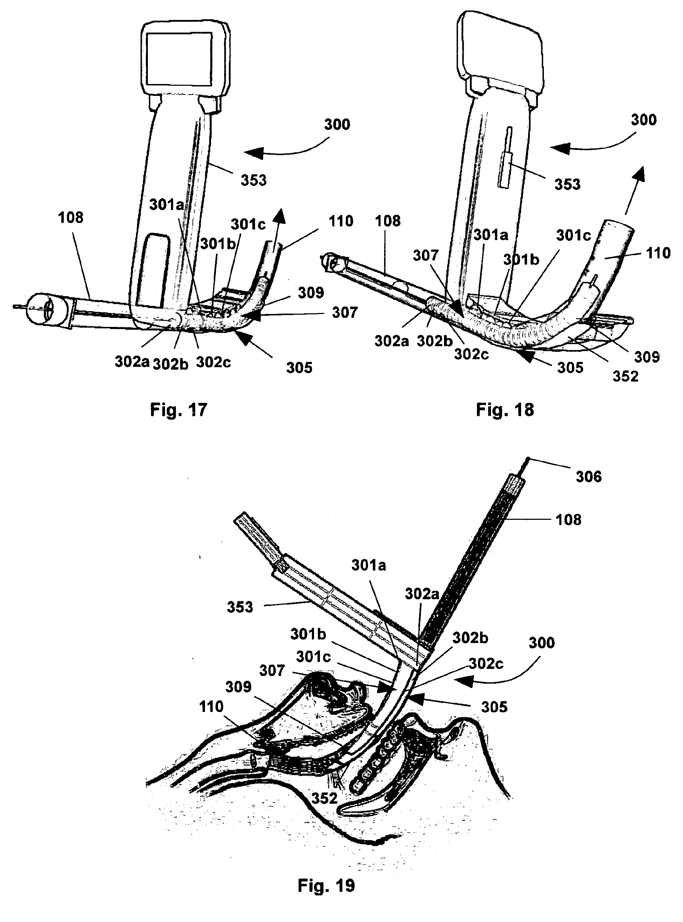

Figures 17 and 18, show according to two different angles, another alternative embodiment of the present invention, applied to the blade of a *LMA*® *videolaryngoscope* 300 with magnetic elements, according to concepts and disclosures from the European patent application EP 11425045 (De Domenico), filed the 23 February 2011.

In device 300 the guiding function is achieved through a *magnetic platform* 305, that is placed into the blade, and that interacts with a *magnetic train* 307, that has been previously and steadily inserted within an endotracheal tube 108.

The *magnetic platform* 305 comprises a first set of magnetic elements 301 a, 301 b, ..., etc., that are all connected each other, by a first flexible supporting structure; the *magnetic train* 307 comprises instead a second set of magnetic elements 302a, 302b, ..., etc., that are all connected each other, by a second flexible supporting structure.

The elements 302a, 302b, ..., etc., of the *magnetic train* 307 have an opposite polarity in respect of elements 301 a, 301 b, ... , etc., of said *magnetic platform* 305, so that a magnetic attractive force is generated by interaction all the time, keeping said endotracheal tube 108 strictly in contact with said *magnetic platform* 305.

An operator can move the endotracheal tube 108, forward or backward, along the longitudinal profile of the laryngoscope's blade, in order to achieve a guiding and routing system of the same endotracheal tube 108, because the tube follows a respective curved trajectory and it extends itself with its far end 110 aligned to a direction that follows the tangent to said laryngoscope's profile, at the last point of magnetic contact 309.

Therefore, the guiding system of the present invention is integrated in said magnetic guiding system, already known from the prior art, and it includes a mobile protruding wall 352, that is pushed outside and smoothly forward, through a longitudinal groove, from the blade of the same device 300.

A forward motion of sliding button 353 activates a mechanism, which is smooth or by click, pushing outside and forward the protruding element 353. In such a way, elements 352 enable the tracheal tube 108 to "play off the cushion", because the far end 110 can be curved upwards, smoothly and in an adjustable way, and can be addressed to trachea of a patient, as illustrated in figure 19.

The supporting device, subject of the present invention, can be easily integrated with optical means, typical of a *Airtraq*® *videolaryngoscope,* or a *LMA*® *videolaryngoscope,* or other similar instruments.

In example, the housing containing the mechanisms that push said protruding element outside, can be perfectly parallel to a visual optical channel, through which the visual signals of the detected image are transmitted, from the far end of the laryngoscope.

Furthermore, some embodiments of the invention disclose that said operative channel can be placed at the right side, from the point of view of an operator, or at the left side. Then, another embodiment of invention discloses that said operative channel, along which said endotracheal tube 108 is inserted, can be central and parallel in respect to the two service channels, one of them being in example an optical visual channel, and the other the one where said protruding element is contained.

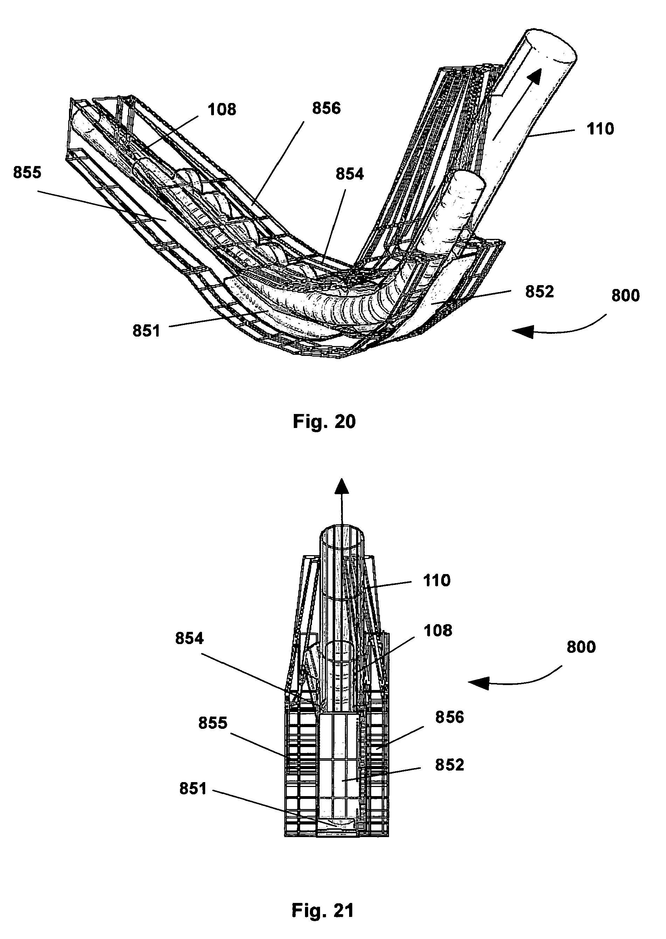

Furthermore, according to another embodiment 800 of the present invention, shown in figures 20 and 21, the operative channel 854, along which the endotracheal tube 108 is inserted, can be placed centrally in respect to two lateral channels 855, 856, each of them being divided at least in two semi-channels. These lateral semi-channels are particularly suitable as respective housings of two sets of protruding elements 851, 852 (one on the right and one on the left), with the other two semi-channels being used: one to contain an optical visual system, the other one to contain any other device that can be, in example, a magnetic platform, or a more simple rod that, being placed under one of the semi-channels containing the protruding elements 851, 852 (and ending with the distal part close to the far end of blade) is able to curve/sustain the far end of blade, achieving the same function of a McCoy laryngoscope.

In such a way, the patient's anatomical parts result to be better protected, in respect to situations where the endotracheal tube 108 could touch the tongue and tissues, with risk of abrasions and inner scratching for the patient.

Therefore, the above examples show that the present invention achieves all the proposed objectives. In particular, it permits to obtain a device that permits to overcome all the drawbacks of the prior art, achieving a great versatility of use, because it can be embedded into large part of the available systems supporting the orotracheal intubation, actually on the market, like i.e.: laryngoscopes, videolaryngoscopes, tunneled videolaryngoscopes, etc.

In particular, the device of this invention achieves a guiding and routing system for an endotracheal tube by use of one or more planar elements, each having a profile that is perfectly adapted to the curvature of said blade in which it is placed, and from which it can protrude out, on command, through a groove, in order to achieve one or more respective fixed transversal wall.

Another significant feature of the present invention is the possibility of integration in a guiding system by magnetic means, according to concepts and disclosure from the European patent application EP 11425045 (De Domenico), so that the intubation procedure can be adapted to different operative circumstances, especially in case where a direct or indirect visual perception of the situation is possible. More specifically, the above integration would achieve a synergy of the two systems, the first based on planar elements and the second based on magnetic elements, so that a smaller size of both devices is possible, according to smaller planar elements and smaller magnetic elements either, without affecting the effectiveness of instruments, and improving instead the general efficiency due to the decreased length of inter-foreteeth distance.

Then, the disclosed device permits therefore to achieve a significant decrease of thickness, a variable routing direction at each step of the procedure, under control of a guiding mechanism, a more comfortable intubation that does not require any use of a stylet to define shape and profile.

The present invention has been described for illustrative but not limitative purposes, according to its preferred embodiments, but it is clear that modifications and/or changes can be introduced by those skilled in the art without departing from the relevant scope, as defined in the enclosed claims.

## Claims

1. Device supporting a tracheal tube, preferably applied to a blade (104) of a laryngoscope, or an *Airtraq*® *videolaryngoscope* (100), or a *LMA*® *videolaryngoscope,* or other similar instruments, particularly suitable as a support to procedure of patient's tracheal intubation, and **characterized in that** comprising:
- one or more planar elements (151), (152), each of them having a profile that is perfectly adapted to the curvature of said blade (104) in which it is placed, and from which it can protrude out, on command, through a longitudinal groove, in order to achieve one or more fixed transversal walls, that are continuous or in patches; a tracheal tube (108) that goes with its far end (110) across said one or more transversal walls, and that can be curved and addressed towards trachea of a patient;
- a set of mechanical gears that: according to a back position of a sliding button (153), keep said one or more planar elements (151), (152), placed inside the above blade (104); and that according to a front position of the same sliding button (153), push outside and distally the above said one or more planar elements (151), (152), in order to achieve said one or more fixed transversal walls.

2. Device supporting a tracheal tube, according to previous claim, **characterized in that**:
- the mechanism that allows a protruding element (607) to be pushed out, on command, through a longitudinal groove of blade (606), includes a triangle-based support (605); this support (605) can move laterally outside of blade (606), following a motion transmitted by a rod (601) connected to it; in the upper part, the same rod (601) is connected to a sliding button;
- a motion forward of said button causes the motion forward of rod (601), following the direction of arrow and, through an articulated tract (603) and pivots (602) and (604), the same motion is transmitted to support (605), sliding outside of blade (606), along a lateral oblique wall, in touch to another triangle-based and fixed element (610); a protruding element (607) is connected on the top of said support (605), and represents a wall that, touched by the far end of a tracheal tube, enables it to "play off the cushion", changing and curving the insertion trajectory of the same tracheal tube.

3. Device supporting a tracheal tube, according to previous claim 2, **characterized in that**:
- the protruding element (607) has a transversal edge (609) on the inner side, that is intercepted by a respective edge (608) on the profile of blade (606), so that an end-run function is embedded in motion of said element (607).

4. Device supporting a tracheal tube, according to previous claim 2, **characterized in that**:
- said mechanism pushing outside the protruding element (607) is perfectly reversible, and that means that a motion backward of said sliding button causes a motion backward of said rod (601) following a direction opposite to arrow and, through the same articulated tract (603) and pivots (602) and (604), the motion is transmitted to support (605), sliding inside of blade (606), along a lateral oblique wall, in touch to the triangle-based and fixed element (610).

5. Device supporting a tracheal tube, according to one or more of previous claims, **characterized in that**:
- said planar elements (151), (152), are preferably made using a material, or a metallic alloy, that is highly elastic and strong, like in example a flexible steel.

6. Device supporting a tracheal tube, according to one or more of previous claims, **characterized in that**:
- said device is integrated with a magnetic guiding system, according to concepts and disclosures from the European patent application EP 11425045 (De Domenico), and in laryngoscope (200), or in *Airtraq*® *videolaryngoscope* (100), or in a *LMA*® *videolaryngoscope* (300), or in other similar instruments, the guiding function is achieved through a *magnetic platform* (205), that is placed into the blade, and that interacts with a *magnetic train* (207), that has been previously and steadily inserted within an endotracheal tube (108).

7. Device supporting a tracheal tube, according to previous claim 6, **characterized in that**:
- The *magnetic platform* (205) comprises a first set of magnetic elements (201 a), (201 b), ..., etc., that are all connected each other, by a first flexible supporting structure; the *magnetic train* (207) comprises instead a second set of magnetic elements (202a), (202b), ..., etc., that are all connected each other, by a second flexible supporting structure;
- the elements (202a), (202b), ..., etc., of the *magnetic train* (207) have an opposite polarity in respect of elements (201 a), (201 b), ... , etc., of said *magnetic platform* (205), so that a magnetic attractive force is generated by interaction all the time, keeping said endotracheal tube (108) strictly in contact with said *magnetic platform* (205),
so that an operator can move the endotracheal tube (108), forward or backward, along the longitudinal profile of said operative channel (204), in order to achieve a guiding and routing system of the same endotracheal tube (108), because the tube follows a respective curved trajectory and it extends itself with its far end (110) aligned to a direction that follows the tangent to said operative channel's (204) profile, at the last point of magnetic contact (209).

8. Device supporting a tracheal tube, according to previous claim 1, preferably applied to a blade (706) of a laryngoscope, **characterized in that** comprising as an alternative:
- a protruding element (707) composed, indeed, by a unique flexible plate, having a set of small cylinders (701 a), (701 b), (701c),..., etc., installed on it following a longitudinal line, so that when they intercept the edge (708) embed the same function of end-run, as previously described in claim 3.

9. Device supporting a tracheal tube, according to previous claim 8, **characterized in that**:
- a protruding element (709) is represented by a unique spoon-shaped convex item; again, a set of small cylinders (701a), (701 b), (701c),..., etc., are installed on it following a longitudinal line, and when they intercept the edges (708) and (710) embed the same function of end-run, as previously described in claim 3.

10. Device supporting a tracheal tube, according to one or more of previous claims, **characterized in that**:
- the mechanism controlling the above action of pushing said protruding elements (151), (152), or (707) or (709), outside, or pulling them inside of blade, and/or their simultaneous motion forward or backward along the longitudinal axis of the same blade, can further include additional gears, defining and enabling a "click" mechanism; these additional gears can include springs that are charged, with stopping clips that, when removed by a command button, cause the protruding elements (151), (152), or (707) or (709), to be immediately "clicked" outside, or "clicked" inside.

11. Device supporting a tracheal tube, according to one or more of previous claims, **characterized in that**:
- said device can include more mechanisms, one for each of said protruding elements (151), (152), that can be activated simultaneously, or sequentially, in order to obtain a medical device that is comfortable and easy to be used.

12. Device supporting a tracheal tube, according to one or more of previous claims, **characterized in that**:
- the housing containing the mechanisms that push said protruding elements (151), (152), or (707) or (709), outside, is perfectly parallel to a visual optical channel, through which the visual signals of the detected image are transmitted, from the far end of a *Airtraq*® *videolaryngoscope* (100), or *LMA*® *videolaryngoscope* (300), or other similar instruments.

13. Device supporting a tracheal tube, according to previous claim 12, **characterized in that**:
- said housing containing the mechanisms that push said protruding elements (151), (152), or (707) or (709), outside, is placed at the right side, from the point of view of an operator, or at the left side.

14. Device supporting a tracheal tube, according to one or more of previous claims, **characterized in that**:
- the operative channel, along which said endotracheal tube (108) is inserted, is central and parallel in respect to the two service channels, one of them being in example an optical visual channel, and the other the one where one of said protruding elements (151), (152), or (707) or (709), is contained,
so that the patient's anatomical parts result to be better protected, in respect to situations where the endotracheal tube (108) could touch the tongue and tissues, with risk of abrasions and inner scratching for the patient.

15. Device supporting a tracheal tube, according to one or more of previous claims, **characterized in that**:
- the operative channel (854), along which the endotracheal tube (108) is inserted, is placed centrally in respect to two lateral channels (855), (856), each of them being divided at least in two semi-channels; these lateral semi-channels are particularly suitable as respective housings of two sets of protruding elements (851), (852) - one on the right and one on the left - with the other two semi-channels being used: one to contain an optical visual system, the other one to contain any other device that can be, in example, a magnetic platform, or a more simple rod that, being placed under one of the semi-channels containing the protruding elements (851), (852) - and ending with the distal part close to the far end of blade - is able to curve/sustain the far end of blade, achieving the same function of a McCoy laryngoscope.
